# EUROPÄISCHE PATENTANMELDUNG

(11) **EP 1 669 386 A1**
(43) Veröffentlichungstag der Anmeldung: **14.06.2006**
(21) Anmeldenummer: 04028865.6
(22) Anmeldetag: 06.12.2004
(51) Int. Cl.: C08G 61/10, C08G 61/02, C07C 43/225, C07C 211/56, C07C 25/22

(54) **Teilkonjugierte Polymere, deren Darstellung und Verwendung**

(71) Anmelder: Covion Organic Semiconductors GmbH, 65926 Frankfurt am Main (DE)
(72) Erfinder: Heun, Susanne Dr., 65812 Bad Soden (DE); Parham, Amir Dr., 65929 Frankfurt (DE); Falcou, Aurélie Dr., 60322 Frankfurt (DE)

(57) **Zusammenfassung**

Die vorliegende Erfindung betrifft teilkonjugierte Spirobifluoren-Polymere, die über beide Spiro-Molekülhälften im Polymer verknüpft sind. Die erfindungsgemäßen Materialien zeigen reinere Emissionsfarbe und höhere Lebensdauer als Materialien gemäß dem Stand der Technik und eignen sich daher besser zur Verwendung in polymeren organischen Leuchtdioden.

## Beschreibung

Seit einigen Jahren läuft eine breit angelegte Forschung zur Kommerzialisierung von Anzeige- und Beleuchtungselementen auf Basis polymerer (organischer) Leuchtdioden (PLEDs). Ausgelöst wurde diese Entwicklung durch die Grundlagenentwicklungen, welche in WO 90/13148 offenbart sind. Seit kurzem sind auch erste, einfache Produkte (kleine Anzeigen in einem Rasierapparat und einem Möbiltelefon der Fa. PHILIPS N.V.) auf dem Markt erhältlich. Allerdings sind immer noch deutliche Verbesserungen der verwendeten Materialien nötig, um diese Displays zu einer echten Konkurrenz zu den derzeit marktbeherrschenden Flüssigkristallanzeigen (LCD) zu machen. Dabei ist es für die Erzeugung aller drei Emissionsfarben nötig, bestimmte Comonomere in die entsprechenden Polymere einzupolymerisieren (vgl. z. B. WO 00/46321, WO 03/020790 und WO 02/077060). So ist dann in der Regel, ausgehend von einem blau emittierenden Grundpolymer ("backbone"), die Erzeugung der beiden anderen Primärfarben Rot und Grün möglich.

Die konjugierten Polymere gemäß dem Stand der Technik zeigen zum Teil schon gute Eigenschaften in der Anwendung in PLEDs. Trotz der bereits erzielten Fortschritte entsprechen diese Polymere allerdings noch nicht den Anforderungen, die an sie für hochwertige Anwendungen gestellt werden. Insbesondere ist die Lebensdauer der grün und vor allem der blau emittierenden Polymere für viele Anwendungen noch nicht ausreichend.

Ohne an eine bestimmte Theorie gebunden sein zu wollen, vermuten wir, dass die elektronischen Eigenschaften der konjugierten Polymere noch nicht optimal für einen ausbalancierten Ladungstransport in der elektronischen Vorrichtung sind. Dadurch ist teilweise der Elektronen- bzw. der Lochstrom in der Vorrichtung zu groß, und es kann sich kein ausbalanciertes Ladungsgleichgewicht einstellen. Ein weiterer Nachteil ist die statistische breite Verteilung effektiver Konjugationslängen im Polymer, die zu Polymerabschnitten undefinierter Länge und relativ breitbandiger Emission und damit geringer Farbreinheit führt. Außerdem kann es für manche Anwendungen sinnvoll sein, die Funktionalitäten der unterschiedlichen Einheiten im Polymer, also beispielsweise Ladungstransport und Emission, voneinander zu trennen. Dies ist in vollständig konjugierten Polymeren nur dadurch möglich, dass beispielsweise die Ladungstransporteinheiten in die Seitenkette des Polymers eingebaut werden, wie in EP 1263834 beschrieben. Diese Funktionen in der Seitenkette sind dann jedoch nicht konjugiert und weisen auch keine längeren konjugierten Abschnitte außer der funktionellen Einheit selber auf, was wiederum für einen guten Ladungstransport nicht immer ausreicht.

In WO 97/20877 und in EP 0882082 werden teilkonjugierte Spirobifluoren-Polymere beschrieben, die über die 2,2'-Positionen der Spirobifluoren-Einheiten verknüpft sind. Als Vorteile dieser Polymere wird vor allem die exzellente Verarbeitbarkeit aufgeführt, weniger jedoch besonders geeignete elektronische Eigenschaften. So wird nur berichtet, dass beim Anlegen einer genügend hohen Spannung Elektrolumineszenz beobachtet wird, ohne eine Aussage über Spannung, Effizienz und Lebensdauer zu treffen. Auch in einigen Publikationen wird die Verwendung von 2,2'-verknüpften Spirobifluoren-Einheiten beschrieben, z. B. von R. D. Miller *et al.* (*Polymer Preprints* **2002**, *43*, 116-117), F.-I. Wu *et al.* (*J. Mater. Chem.* **2002**, *12*, 2893-2897) und B. Huang *et al.* (*Chem. Lett*. **2004,** *33*, 1376-1377). In keiner dieser Publikationen lässt sich jedoch eine besonders gute Eignung dieser Polymere für organische elektrolumineszente Vorrichtungen erkennen.

Es wurde nun überraschend gefunden, dass teilkonjugierte Polymere, die über die beiden nicht miteinander konjugierten Hälften von Spirobifluoren, jedoch über andere als die 2,2'-Positionen, verknüpft sind, sehr gute und den Stand der Technik übertreffende Eigenschaften aufweisen. Diese Polymere zeigen insbesondere bessere Eigenschaften in der Elektrolumineszenz als vergleichbare Polymere, die über die 2,2'-Positionen des Spirobifluorens verknüpft sind. Dies bezieht sich insbesondere auf die Lebensdauer, aber auch auf die Emissionsfarbe, die Strom-Spannungskurven und die Effizienz der Polymere. Dies ist ein überraschendes und unerwartetes Ergebnis. Diese Polymere und deren Verwendung in organischen elektronischen Vorrichtungen sind daher Gegenstand der vorliegenden Erfindung.

Gegenstand der Erfindung sind Polymere, enthaltend mindestens 1 mol%, bevorzugt mindestens 5 mol%, besonders bevorzugt mindestens 10 mol% einer ersten Wiederholeinheit gemäß Formel (1), 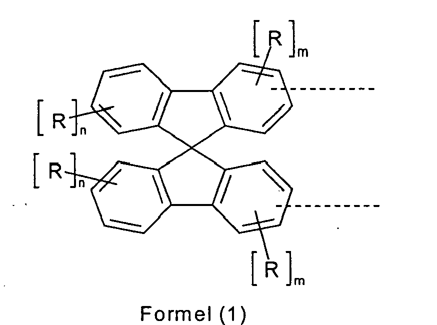 wobei die verwendeten Symbole und Indizes die folgende Bedeutung besitzen:
- R: ist bei jedem Auftreten gleich oder verschieden F, Cl, Br, I, CN, NO₂, OH, N(R¹)₂, Si(R¹)₃, B(R¹)₂, eine geradkettige, verzweigte oder cyclische Alkyl-, Alkoxy- oder Thioalkoxygruppe mit 1 bis 40 C-Atomen, in der auch ein oder mehrere nicht benachbarte C-Atome durch -CR¹=CR¹-, -C≡C-, -NR¹-, -O-, -S-, -CO-O- oder -O-CO-O- ersetzt sein können und wobei auch ein oder mehrere H-Atome durch Fluor ersetzt sein können, ein aromatisches oder heteroaromatisches Ringsystem mit 2 bis 40 C-Atomen, welches auch durch einen oder mehrere nicht-aromatische Reste R substituiert sein kann; oder eine Kombination aus 2, 3, 4 oder 5 der oben genannten Gruppen; dabei können auch zwei oder mehrere der Reste R miteinander ein aliphatisches oder aromatisches, mono- oder polycyclisches Ringsystem bilden;
- R¹: ist bei jedem Auftreten gleich oder verschieden H oder ein aliphatischer oder aromatischer Kohlenwasserstoffrest mit 1 bis 20 C-Atomen;
- n: ist bei jedem Auftreten gleich oder verschieden 0, 1, 2, 3 oder 4;
- m: ist bei jedem Auftreten gleich oder verschieden 0, 1, 2 oder 3;
die gestrichelten Bindungen deuten dabei die Verknüpfung im Polymer an; mit der Maßgabe, dass die Verknüpfung nicht über die 2,2'-Positionen erfolgt; und enthaltend mindestens 1 mol% einer zweiten Wiederholeinheit, die entweder gleich einer Wiederholeinheit gemäß Formel (1) ist oder verschieden ist..

Die Positionen am Spirobifluoren sind im Folgenden der Übersichtlichkeit halber aufgeführt: 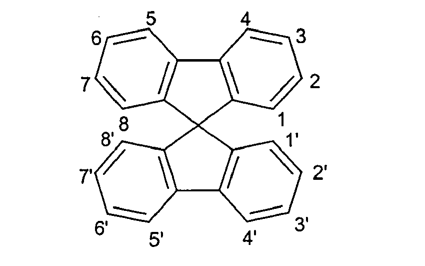 Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, n-Pentyl, s-Pentyl, Cyclopentyl, n-Hexyl, Cyclohexyl, n-Octyl, Cyclooctyl, 2-Ethylhexyl, 4-Ethylhexyl, 1,1,5-Trimethylheptyl, 1-Ethyl-1,5-dimethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Octenyl, Cyclooctenyl, Ethinyl, Propinyl, Butinyl, Pentinyl, Hexinyl oder Octinyl verstanden. Unter einer C₁- bis C₄₀-Alkoxygruppe werden besonders bevorzugt Methoxy, Ethoxy, n-Propoxy, i-Propoxy, n-Butoxy, i-Butoxy, s-Butoxy, 2-Methylbutoxy oder t-Butoxy verstanden. Unter einem C₂- bis C₄₀-aromatischen oder heteroaromatischen Ringsystem werden besonders bevorzugt Phenyl, Biphenyl, Naphthyl, Anthracenyl, Triphenylenyl, [1,1';3',1"]-Terphenyl-2'-yl, Binaphthyl, Phenanthrenyl, Fluorenyl, Spirobifluorenyl, Dihydrophenanthrenyl, Dihydropyrenyl, Tetrahydropyrenyl, 2-Pyridyl, 3-Pyridyl, 4-Pyridyl, 2-Pyrimidyl, 4-Pyrimidyl, 5-Pyrimidyl, 2-Pyrazinyl, 3-Pyridazinyl, 4-Pyridazinyl, 2-Triazinyl, Chinolinyl, Isochinolinyl, Chinoxalinyl, Acridinyl, Benzochinolinyl, Benzoisochinolinyl, 2-Thienyl, 3-Thienyl, 2-Furanyl, 3-Furanyl, 2-Pyrrolyl, 3-Pyrrolyl, Oxadiazolyl, Benzothiadiazolyl, Indolyl, Isoindolyl, Pentafluorophenyl, Tetrafluorophenyl, 3,5-Bistrifluoromethylphenyl, Heptafluoronaphthyl, o-Tolyl, m-Tolyl, p-Tolyl, 2,6-Dimethylphenyl, 2,6-Diethylphenyl, 2,6-Di-i-propylphenyl, 2,6-Di-t-butylphenyl, o-t-Butylphenyl, m-t-Butylphenyl oder p-t-Butylphenyl verstanden, die auch durch die oben genannten Reste R¹ substituiert sein können.

Auch wenn dies aus der Beschreibung hervorgeht, sei hier nochmals explizit darauf verwiesen, dass die Struktureinheiten gemäß Formel (1) unsymmetrisch substituiert sein können, d. h. dass in einer Einheit unterschiedliche Substituenten R vorhanden sein können, bzw. diese auch an unterschiedlichen Positionen gebunden sein können.

Unter einem aromatischen bzw. heteroaromatischen Ringsystem im Sinne dieser Erfindung soll ein System verstanden werden, das nicht notwendigerweise nur aromatische bzw. heteroaromatische Gruppen enthält, sondern in dem auch mehrere aromatische bzw. heteroaromatische Gruppen durch eine kurze nicht-aromatische Einheit (< 10 % der von H verschiedenen Atome, bevorzugt < 5 % der von H verschiedenen Atome), wie beispielsweise sp³-hybridisierter C, O, N, etc., unterbrochen sein können. So sollen also beispielsweise auch Systeme wie 9,9'-Spirobifluoren, 9,9-Diarylfluoren, Triarylamin, etc. als aromatische Systeme im Sinne dieser Erfindung verstanden werden. Außerdem kann ein solches aromatisches bzw. heteroaromatisches Ringsystem auch in einem ansonsten nichtkonjugierten Polymer (z. B. in einer Polystyrolkette mit definierten konjugierten Segmenten) vorliegen.

Einheiten gemäß Formel (1) liegen in zwei enantiomeren Formen vor. Dabei ist es gleichermaßen erfindungsgemäß, das Racemat, also die 1 : 1 Mischung der beiden Enantiomeren, oder eines der beiden Enantiomeren in angereicherter oder isolierter Form zu verwenden.

Bevorzugt sind Einheiten gemäß Formel (1), in denen für die Symbole und Indizes gilt:
- R: ist bei jedem Auftreten gleich oder verschieden F, CN, NO₂, OH, N(R¹)₂, B(R¹)₂, eine geradkettige, verzweigte oder cyclische Alkyl-, Alkoxy- oder Thioalkoxygruppe mit 1 bis 20 C-Atomen, in der auch ein oder mehrere nicht benachbarte C-Atome durch -CR¹=CR¹-, -C≡C-, -NR¹-, -O-, -S- oder -CO-O-ersetzt sein können und wobei auch ein oder mehrere H-Atome durch Fluor ersetzt sein können, ein aromatisches oder heteroaromatisches Ringsystem mit 3 bis 30 C-Atomen, welches auch durch einen oder mehrere nicht-aromatische Reste R substituiert sein kann; oder eine Kombination aus 2, 3 oder 4 der oben genannten Gruppen; dabei können auch zwei oder mehrere der Reste R miteinander ein aliphatisches oder aromatisches, mono- oder polycyclisches Ringsystem bilden;
- R¹: ist wie oben beschrieben;
- n: ist bei jedem Auftreten gleich oder verschieden 0, 1, 2 oder 3;
- m: ist bei jedem Auftreten gleich oder verschieden 0, 1 oder 2.

Besonders bevorzugt sind Einheiten gemäß Formel (1), in denen für die Symbole und Indizes gilt:
- R: ist bei jedem Auftreten gleich oder verschieden F, N(R¹)₂, eine geradkettige, verzweigte oder cyclische Alkyl- oder Alkoxygruppe mit 1 bis 10 C-Atomen, in der auch ein oder mehrere nicht benachbarte C-Atome durch -CR¹=CR¹-, -C≡C-, -O- oder -CO-O- ersetzt sein können und wobei auch ein oder mehrere H-Atome durch Fluor ersetzt sein können, ein aromatisches oder heteroaromatisches Ringsystem mit 4 bis 20 C-Atomen, welches auch durch einen oder mehrere nicht-aromatische Reste R substituiert sein kann; oder eine Kombination aus 2 oder 3 der oben genannten Gruppen; dabei können auch zwei oder mehrere der Reste R miteinander ein aliphatisches oder aromatisches, mono- oder polycyclisches Ringsystem bilden;
- R¹: ist wie oben beschrieben;
- n: ist bei jedem Auftreten gleich oder verschieden 0, 1 oder 2;
- m: ist bei jedem Auftreten gleich oder verschieden 0 oder 1.

Weiterhin bevorzugt sind Einheiten gemäß Formel (1), die an den beiden Spiro-Molekülhälften symmetrisch substituiert sind. Diese Bevorzugung ist durch die leichtere synthetische Zugänglichkeit der Monomere zu begründen.

Erfindungsgemäß sind verschiedene Verknüpfungen der Einheiten gemäß Formel (1) im Polymer möglich. Bevorzugt ist, wenn die Verknüpfung über gleiche Positionen an den beiden Spiro-Molekülhälften erfolgt. Diese Bevorzugung ergibt sich wiederum aus der leichteren synthetischen Zugänglichkeit der entsprechenden Monomere. Eine bevorzugte Ausführungsform der Erfindung ist daher die Verknüpfung über die 1,1'-Positionen, über die 3,3'-Positionen oder über die 4,4'-Positionen. Eine besonders bevorzugte Ausführungsform der Erfindung ist die Verknüpfung über die 1,1'-Positionen oder über die 4,4'-Positionen. Ganz besonders bevorzugt ist die Verknüpfung über die 4,4'-Positionen.

Aus der Art der Verknüpfung folgt, dass die Einheiten gemäß Formel (1) in die Hauptkette des Polymers gebunden sind und dass die Einheiten gemäß Formel (1) zu einem Knick in der Polymerkette führen, da zwischen den beiden Verknüpfungen zum Polymer das sp³-hybridisierte Spiro-Kohlenstoffatom liegt, das für die orthogonale Verknüpfung der beiden Spiro-Molekülhälften verantwortlich ist.

Die erfindungsgemäßen Polymere können teilkonjugiert oder nicht-konjugiert sein. Vollständig konjugierte Polymere sind hier nicht möglich, da die Einheiten gemäß Formel (1) immer eine Konjugationsunterbrechung darstellen. In einer bevorzugten Ausführungsform der Erfindung handelt es sich um ein teilkonjugiertes Polymer. Besonders bevorzugt enthält das Polymer außer Einheiten gemäß Formel (1) keine weiteren Einheiten, die die Konjugation des Polymers unterbrechen.

Unter einem teilkonjugierten Polymer im Sinne dieser Erfindung soll ein Polymer verstanden werden, in dem längere konjugierte Abschnitte in der Hauptkette durch nicht-konjugierte Abschnitte unterbrochen sind, welche, wie oben erwähnt, bevorzugt durch Einheiten gemäß Formel (1) erzeugt werden. Diese konjugierten Abschnitte werden durch sp²-hybridisierte (oder auch sp-hybridisierte) Kohlenstoffatome erzeugt, die auch durch entsprechende Heteroatome ersetzt sein können, was im einfachsten Fall abwechselndes Vorliegen von Doppel- und Einfachbindungen bedeutet. Des Weiteren wird in diesem Anmeldetext ebenfalls als konjugiert bezeichnet, wenn sich in der Hauptkette beispielsweise Arylamineinheiten und/oder bestimmte Heterocyclen (d. h. Konjugation über N-, O- oder S-Atome) und/oder metallorganische Komplexe (d. h. Konjugation über das Metallatom) befinden. Hingegen würden Einheiten wie beispielsweise einfache Alkylenbrücken, (Thio)Ether-, Ester-, Amid- oder Imidverknüpfungen als nicht-konjugierte Segmente definiert.

Die erfindungsgemäßen Polymere enthalten neben Einheiten gemäß Formel (1) bevorzugt noch weitere Strukturelemente, die verschieden von Einheiten gemäß Formel (1) sind, und sind somit als Copolymere zu bezeichnen. Bevorzugt sind die weiteren Strukturelemente konjugiert. Hier sei vor allem auch auf die relativ umfangreichen Auflistungen in WO 02/077060, in der nicht offen gelegten Anmeldeschrift DE 10337346.2 und die darin aufgeführten Zitate verwiesen. Diese weiteren Struktureinheiten können beispielsweise aus den im Folgenden beschriebenen Klassen stammen:
Gruppe 1: Aromatische Einheiten, welche üblicherweise das Polymer-Grundgerüst darstellen:
   Einheiten dieser Gruppe sind aromatische, carbocyclische Strukturen mit 6 bis 40 C-Atomen, die substituiert oder unsubstituiert sein können, wobei als Substituenten die oben genannten Reste R in Frage kommen. Hier kommen Fluoren-Derivate (z. B. EP 0842208, WO 99/54385, WO 00/22027, WO 00/22026, WO 00/46321) in Betracht. Des Weiteren sind auch Spirobifluoren-Derivate (z. B. EP 0707020, EP 0894107, WO 03/020790) eine Möglichkeit. Auch Polymere, die eine Kombination der beiden erstgenannten Monomer-Einheiten enthalten (WO 02/077060), wurden bereits vorgeschlagen. In der nicht offen gelegten Anmeldung DE 10337346.2 sind Dihydrophenanthren-Derivate beschrieben. Weiterhin kommen cis- oder trans-Indenofluoren-Derivate (z. B. WO 04/041901, EP 03014042.0) in Frage, aber auch 1,4-Phenylen-Derivate, 4,4'-Biphenylylen-Derivate, 4,4"-Terphenylylen-Derivate, 2,7- oder 3,6-Phenanthren-Derivate (z. B. DE 0102004020298.2), Dihydropyren- oder Tetrahydropyren-Derivate und weitere nicht explizit aufgeführte aromatische Strukturen. Einheiten aus Gruppe 1 sind also ausgewählt aus der Gruppe der Fluoren-Derivate, der Spirobifluoren-Derivate, der Dihydrophenanthren-Derivate, der cis- oder trans-Indenofluoren-Derivate, der 1,4-Phenylen-Derivate, der 4,4'-Biphenylen-Derivate, der 4,4"-Terphenylen-Derivate, der 2,7- oder 3,6-Phenanthren-Derivate, der Dihydropyren- oder der Tetrahydropyren-Derivate. Bevorzugte Einheiten aus dieser Gruppe sind ausgewählt aus Spirobifluoren, Fluoren, Dihydrophenanthren, cis-Indenofluoren, trans-Indenofluoren und 2,7-Phenanthren, die durch R substituiert oder unsubstituiert sein können.
Gruppe 2: Einheiten, welche die Morphologie bzw. die Emissionsfarbe verändern:
   Es sind auch Strukturelemente möglich, die die Morphologie, aber auch die Emissionsfarbe der resultierenden Polymere beeinflussen können. Bevorzugt sind diese dabei ausgewählt aus der Gruppe der durch R substituierten oder unsubstituierten kondensierten aromatischen Strukturen mit 6 bis 40 C-Atomen oder auch Tolan-, Stilben- oder Bisstyrylarylenderivaten, wie z. B. 1,4-Naphthylen-, 1,4- oder 9,10-Anthrylen-, 1,6- oder 2,7- oder 4,9-Pyrenylen-, 3,9- oder 3,10-Perylenylen-, 4,4'-Bi-1,1'-naphthylylen-, 4,4'-Tolanylen-, 4,4'-Stilbenylen- oder 4,4"-Bisstyrylarylenderivate.
Gruppe 3: Einheiten, welche die Lochinjektions- und/oder -transporteigenschaften der Polymere erhöhen:
   Dies sind im Allgemeinen aromatische Amine oder Phosphine oder elektronenreiche Heterocyclen und sind bevorzugt ausgewählt aus der Gruppe der durch R substituierten oder unsubstituierten Triarylamine, Benzidine, N,N,N',N'-Tetraaryl-para-phenylendiamine, Triarylphosphine, Phenothiazine, Phenoxazine, Dihydrophenazine, Thianthrene, Dibenzo-*p*-dioxine, Phenoxathiine, Carbazole, Azulene, Thiophene, Pyrrole, Furane und weiteren O-, S- oder N-haltigen Heterocyclen mit hoch liegendem HOMO (HOMO = höchstes besetztes Molekülorbital). Diese Einheiten können in die Hauptkette oder in die Seitenkette des Polymers eingebaut werden. Je nach Struktur ist auch das Polymergrundgerüst in der Lage, ausreichend gut Löcher zu leiten, so dass nicht zwangsläufig Einheiten aus Gruppe 3 anwesend sein müssen.
Gruppe 4: Einheiten, welche die Elektroneninjektions- und/oder -transporteigenschaften der Polymere erhöhen:
   Dies sind im Allgemeinen elektronenarme Aromaten oder Heterocyclen und sind bevorzugt ausgewählt aus der Gruppe der durch R substituierten oder unsubstituierten Pyridine, Pyrimidine, Pyridazine, Pyrazine, Triazine, Oxadiazole, Chinoline, Chinoxaline oder Phenazine, aber auch Verbindungen wie Triarylborane und weiteren O-, S- oder N-haltigen Heterocyclen mit niedrig liegendem LUMO (LUMO = niedrigstes unbesetztes Molekülorbital). Je nach Struktur ist auch das Polymergrundgerüst in der Lage, ausreichend gut Elektronen zu leiten, so dass nicht zwangsläufig Einheiten aus Gruppe 4 vorhanden sein müssen.
Gruppe 5: Einheiten, die Kombinationen von Einzeleinheiten der Gruppe 3 und Gruppe 4 aufweisen:
   Es kann auch bevorzugt sein, wenn in den erfindungsgemäßen Polymeren Einheiten enthalten sind, in denen Strukturen, welche die Lochmobilität und welche die Elektronenmobilität erhöhen, direkt aneinander gebunden sind, also Strukturen aus den oben genannten Gruppen 3 und 4. Viele dieser Einheiten verschieben die Emissionsfarbe ins Grüne, Gelbe oder Rote; ihre Verwendung eignet sich also beispielsweise für die Erzeugung anderer Emissionsfarben aus ursprünglich blau emittierenden Polymeren.
Gruppe 6: Einheiten, die aus dem Triplett-Zustand Licht emittieren Struktureinheiten aus dieser Gruppe können auch bei Raumtemperatur mit hoher Effizienz aus dem Triplettzustand Licht emittieren und zeigen also Elektrophosphoreszenz statt Elektrofluoreszenz. Hierfür eignen sich zunächst Verbindungen, welche Schweratome mit einer Ordnungszahl von mehr als 36 enthalten. Besonders geeignet sind Verbindungen, welche d- oder f-Übergangsmetalle enthalten, die diese Bedingung erfüllen. Ganz besonders bevorzugt sind Struktureinheiten, welche Elemente der Gruppe 8 bis 10 (Ru, Os, Rh, Ir, Pd, Pt) enthalten. Diese Metallkomplexe können in die Hauptkette und/oder in die Seitenkette des Polymers gebunden werden. Als geeignet hat sich auch der Einbau derartiger Metallkomplexe an Verzweigungspunkte im Polymer erwiesen, wie beispielsweise in DE 102004032527.8 beschrieben. Wenn Einheiten aus Gruppe 6 vorhanden sind, kann es bevorzugt sein, wenn gleichzeitig auch Einheiten aus Gruppe 7 vorhanden sind. Auch auch ohne derartige Einheiten aus Gruppe 7 können mit Triplett-Emittern sehr gute Ergebnisse und sehr hohe Effizienzen erreicht werden.
Gruppe 7: Einheiten, welche den Transfer vom Singulett- zum Triplett-Zustand unterstützen
   Für den Einsatz von Triplett-Emittern kann es bevorzugt sein, unterstützend weitere Strukturelemente einzusetzen, welche den Übergang vom Singulett- zum Triplett-Zustand und damit die Elektrophosphoreszenzeigenschaften verbessern. Hierfür kommen beispielsweise Carbazoleinheiten in Frage, wie in WO 04/070772 und DE 10328627.6 beschrieben, aber beispielsweise auch Keto-, Phosphinoxid-, Sulfoxid- oder Sulfon-Einheiten, wie in der nicht offen gelegten Anmeldung DE 10349033.7 beschrieben, oder Silaneinheiten, wie in der nicht offen gelegten Anmeldung DE 102004023278.4 beschrieben. Die Einheiten sind also bevorzugt ausgewählt aus der Gruppe der Carbazoleinheiten, der überbrückten Carbazoleinheiten, der Keto-, Phosphinoxid-, Sulfoxid- oder Sulfon-Einheiten und der Silaneinheiten.

Bevorzugt sind Polymere, die neben Struktureinheiten gemäß Formel (1) zusätzlich noch eine oder mehrere Einheiten ausgewählt aus den Gruppen 1 bis 7 enthalten.

Dabei kann es auch vorteilhaft sein, wenn gleichzeitig mehr als eine Struktureinheit aus einer der Gruppen 1 bis 7 vorliegt.
Besonders bevorzugt sind Polymere, die neben Einheiten gemäß Formel (1) noch Einheiten aus der Gruppe 1 enthalten, ganz besonders bevorzugt mindestens 30 mol% dieser Einheiten.
Besonders bevorzugt sind weiterhin Polymere, die neben Einheiten gemäß Formel (1) noch Einheiten aus der Gruppe 2 und/oder 3 enthalten, ganz besonders bevorzugt mindestens 5 mol% dieser Einheiten.

Die nötige Löslichkeit der Polymere wird v. a. durch die Substituenten an den verschiedenen Wiederholeinheiten gewährleistet, sowohl den Substituenten R und R¹ an Einheiten gemäß Formel (1), wie auch durch Substituenten R an den anderen Wiederholeinheiten.

Bevorzugt ist ein Anteil von 1 - 100 mol% Einheiten gemäß Formel (1). Besonders bevorzugt ist ein Anteil von 5 - 70 mol% Einheiten gemäß Formel (1), ganz besonders bevorzugt ein Anteil von 10 - 50 mol%.

Die erfindungsgemäßen Polymere sind entweder Homopolymere aus Einheiten gemäß Formel (1) oder Copolymere. Erfindungsgemäße Copolymere können dabei neben einer oder mehreren Strukturen gemäß Formel (1) potenziell eine oder mehrere weitere Strukturen aus den oben aufgeführten Gruppen 1 bis 7 besitzen. Dabei kann die Konjugationsunterbrechung durch Einheiten gemäß Formel (1) in Nachbarschaft zu jeder der Struktureinheiten der Gruppen 1 bis 7 erfolgen. Die erfindungsgemäßen Copolymere können statistische, alternierende oder blockartige Strukturen aufweisen oder auch mehrere dieser Strukturen abwechselnd besitzen. Wie Copolymere mit blockartigen Strukturen erhalten werden können, ist beispielsweise in der nicht offen gelegten Anmeldung DE 10337077.3 beschrieben. Ebenso sei an dieser Stelle hervorgehoben, dass das Polymer nicht zwangsläufig linear aufgebaut ist, sondern auch verzweigt sein kann, bzw. auch dendritische Strukturen aufweisen kann.

Die genaue Struktur des Polymers und die exakte Anordnung der Wiederholeinheiten im Polymer ist entscheidend für die Funktion. Ohne an eine bestimmte Theorie gebunden sein zu wollen, vermuten wir, dass in vielen konjugierten Polymeren entweder die Ladungsmobilität für Löcher und/oder die Ladungsmobilität für Elektronen zu hoch ist, so dass sich insgesamt kein ausbalanciertes Ladungsgleichgewicht einstellen kann. Die Folge kann sein, dass ein Ladungsträger unnötig in zu hohem Anteil vorhanden ist, was wiederum zu Temperaturerhöhung durch hohe Ströme und Nebenreaktionen im Polymer oder im Device führen könnte, wodurch sich das Polymer zersetzt und die Lebensdauer der Vorrichtung sinkt. Durch einen schlecht ausbalancierten Ladungstransport ist außerdem die Effizienz niedriger, als sie es bei ausgeglichenem Loch- und Elektronentransport sein könnte. Durch Einführung der erfindungsgemäßen Einheiten gemäß Formel (1) und den gezielten Einbau der einzelnen Funktionalitäten in das Polymer kann diesem Problem abgeholfen werden: Durch Limitierung des Ladungstransports durch die Einheiten gemäß Formel (1), die als Konjugationsunterbrechung eingebaut werden, kann die Ladungsträgermobilität für die entsprechenden Ladungsträger gezielt reduziert und damit die Ladungsbalance in der elektronischen Vorrichtung verbessert werden. Der gezielte Aufbau derartiger Strukturen kann beispielsweise durch Kupplung gemäß Suzuki erfolgen, wobei durch die selektive Reaktion von Halogenen mit Boronsäurederivaten die Anordnung der Monomere genau kontrolliert werden kann.
Ein derartiger Aufbau, in dem die emittierenden Abschnitte des Polymers zwischen zwei Einheiten gemäß Formel (1) limitiert sind, hat weiterhin den Vorteil, dass hier die Konjugationslänge definiert aufgebaut werden kann, was wiederum zu einer weniger breitbandigen Emission und dadurch zu größerer Farbreinheit führt. Insbesondere für Polymere, die aus dem Triplett-Zustand Licht emittieren, scheint es geeignet zu sein, nicht vollständig konjugierte Polymer zu verwenden. Ohne an eine bestimmte Theorie gebunden sein zu wollen, vermuten wir, dass in konjugierten Polymeren der Energieübertrag auf den Triplett-Emitter häufig nicht vollständig ist, bzw. dass ein Rücktransfer vom Triplett-Emitter auf das Polymer stattfindet. Dieses Problem kann umgangen werden, indem Einheiten gemäß Formel (1) in das Polymer eingebaut werden.

Die erfindungsgemäßen Polymere weisen im Allgemeinen 10 bis 10000, bevorzugt 20 bis 5000, besonders bevorzugt 50 bis 2000 Wiederholeinheiten auf.

Die erfindungsgemäßen Polymere werden in der Regel durch Polymerisation von einer oder mehreren Monomersorten hergestellt, von denen mindestens ein Monomer im Polymer zu Einheiten gemäß Formel (1) führt. Entsprechende Polymerisationsreaktionen gibt es prinzipiell viele. Es haben sich hier jedoch einige Typen besonders bewährt, die zu C-C- bzw. zu C-N-Verknüpfungen führen:
(A) Polymerisation gemäß Suzuki;
(B) Polymerisation gemäß Yamamoto;
(C) Polymerisation gemäß Stille;
(D) Polymerisation gemäß Hartwig-Buchwald.

Wie die Polymerisation nach diesen Methoden durchgeführt werden kann und wie die Polymere vom Reaktionsmedium abgetrennt und gereinigt werden können, ist beispielsweise in WO 03/048225 und in WO 04/022626 beschrieben. Wie besonders reine Polymere erhalten werden können, ist beispielsweise in EP 04023475.9 beschrieben.

Zur Synthese der Polymere werden die entsprechenden Monomere benötigt. Für die Synthese von Einheiten aus Gruppe 1 bis 7 sei auf die oben genannte Literatur verwiesen.

Monomere, die in erfindungsgemäßen Polymeren zu Struktureinheiten gemäß Formel (1) führen, sind entsprechende Spirobifluoren-Derivate, die an geeigneten Positionen an beiden Molekülhälften, insbesondere in der 4,4'-Position oder in der 1,1'-Position, geeignete Funktionalitäten aufweisen, die es erlauben, diese Monomereinheit in das Polymer einzubauen. Besonders geeignete Funktionalitäten für die Polymerisation sind Halogene, insbesondere Brom, oder Sulfonate oder für die Polymerisation gemäß Suzuki auch Boronsäurederivate oder für die Polymerisation gemäß Stille auch Trialkylzinnderivate. Die Synthese von 4,4'-Dibromspirobifluoren-Derivaten wurde von X. Cheng *et al.* (*Org. Lett.* **2004**, *6*, 2381-2383) beschrieben. Diese Synthese ermöglicht auch die Einführung von Substituenten in der 1,1'-Position. Aus den Brom-Derivaten lassen sich durch Standard-Reaktionen, wie sie dem Fachmann der organischen Synthese geläufig sind, ebenfalls die Boronsäure-Derivate erzeugen.
Ebenso lassen sich aus den oben beschriebenen 4,4'-Dibrom-1,1'-dialkoxy-Derivaten entsprechende Monomere für die Polymerisation über die 1,1'-Positionen erzeugen. Dafür werden zunächst die 4,4'-Positionen durch Umsetzung des Broms blockiert (beispielsweise durch Kupplung mit einem Boronsäurederivat oder durch Reduktion). Die Alkoxygruppen werden in reaktive funktionelle Gruppen umgewandelt, beispielsweise in Triflatgruppen, die entweder direkt in der Polymerisation eingesetzt werden können oder die beispielsweise in einer palladiumkatalysierten Reaktion in ein Boronsäurederivat umgewandelt werden können.

Ein weiterer Gegenstand der Erfindung sind bifunktionelle monomere Verbindungen gemäß Formel (2), 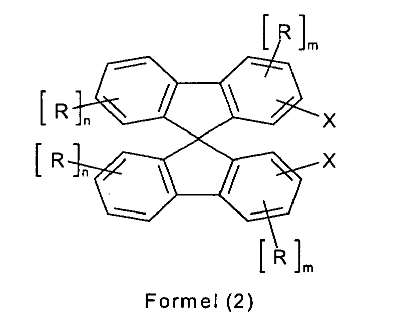 wobei R¹, n und m dieselbe Bedeutung haben, wie oben beschrieben, und weiterhin gilt:
- X: ist bei jedem Auftreten gleich oder verschieden eine Gruppe, die unter Bedingungen der C-C- bzw. C-N-Verknüpfung copolymerisiert;
- R: ist bei jedem Auftreten gleich oder verschieden CN, NO₂, OH, N(R¹)₂, Si(R¹)₃, B(R¹)₂, eine geradkettige, verzweigte oder cyclische Alkyl-, Alkoxy- oder Thioalkoxygruppe mit 1 bis 40 C-Atomen, in der auch ein oder mehrere nicht benachbarte C-Atome durch -CR¹=CR¹-, -C≡C-, -NR¹-, -O-, -S-, -CO-O- oder -O-CO-O- ersetzt sein können und wobei auch ein oder mehrere H-Atome durch Fluor ersetzt sein können, ein aromatisches oder heteroaromatisches Ringsystem mit 2 bis 40 C-Atomen, welches auch durch einen oder mehrere nicht-aromatische Reste R substituiert sein kann; oder eine Kombination aus 2, 3, 4 oder 5 der oben genannten Gruppen; dabei können auch zwei oder mehrere der Reste R miteinander ein aliphatisches oder aromatisches, mono-oder polycyclisches Ringsystem bilden;
wobei die folgende Verbindung von der Erfindung ausgeschlossen ist: 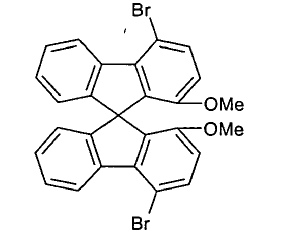 und weiterhin mit der Maßgabe, dass die beiden Gruppen X nicht in 2 und 2'-Position gebunden sind.

Bevorzugt ist X ausgesucht aus Cl, Br, I, O-Tosylat, O-Triflat, O-SO₂R¹, B(OR¹)₂ und Sn(R¹)₃, besonders bevorzugt aus Br, O-Triflat und B(OR¹)₂, wobei R¹ dieselbe Bedeutung hat, wie oben beschrieben, und wobei zwei oder mehr Reste R¹ auch miteinander ein Ringsystem bilden können.

Es kann außerdem bevorzugt sein, das erfindungsgemäße Polymer nicht als Reinsubstanz, sondern als Mischung (Blend) zusammen mit weiteren beliebigen polymeren, oligomeren, dendritischen oder niedermolekularen Substanzen zu verwenden. Diese können beispielsweise die elektronischen Eigenschaften verbessern oder selber emittieren. So ist beispielsweise eine bevorzugte Ausführungsform der Erfindung das Zumischen einer Verbindung, die bei Raumtemperatur mit hoher Effizienz aus dem Triplett-Zustand Licht emittieren kann, so dass dann die Mischung befähigt ist, mit hoher Effizienz aus dem Triplett-Zustand Licht zu emittieren. Derartige Verbindungen sind oben bereits als weitere Strukturelemente für das Polymer beschrieben. Für die zugemischten Verbindungen gelten dieselben Bevorzugungen, wie oben bereits beschrieben. Aber auch elektronisch inerte Blendbestandteile können sinnvoll sein, um beispielsweise die Viskosität einer Lösung oder die Morphologie des gebildeten Films zu kontrollieren. Solche Blends sind daher auch Bestandteil der vorliegenden Erfindung.

Gegenstand der Erfindung sind weiterhin Lösungen und Formulierungen aus einem oder mehreren erfindungsgemäßen Polymeren oder Blends in einem oder mehreren Lösungsmitteln. Wie Polymerlösungen hergestellt werden können, ist beispielsweise in WO 02/072714, WO 03/019694 und der darin zitierten Literatur beschrieben. Diese Lösungen können verwendet werden, um dünne Polymerschichten herzustellen, zum Beispiel durch Flächenbeschichtungsverfahren (z. B. Spin-coating) oder durch Druckverfahren (z. B. InkJet Printing).

Die erfindungsgemäßen Polymere und Blends können in PLEDs verwendet werden. Wie PLEDs hergestellt werden können, wird als allgemeines Verfahren ausführlich in WO 04/037887 beschrieben, das entsprechend für den Einzelfall anzupassen ist. Wie oben beschrieben, eignen sich die erfindungsgemäßen Polymere ganz besonders als Elektrolumineszenzmaterialien in derart hergestellten PLEDs oder Displays.

Als Elektrolumineszenzmaterialien im Sinne der Erfindung gelten Materialien, die als aktive Schicht in einer PLED Verwendung finden können. Aktive Schicht bedeutet, dass die Schicht befähigt ist, bei Anlegen eines elektrischen Feldes Licht abzustrahlen (lichtemittierende Schicht) und/oder dass sie die Injektion und/oder den Transport von positiven und/oder negativen Ladungen verbessert (Ladungsinjektions- oder Ladungstransportschicht).

Gegenstand der Erfindung ist daher auch die Verwendung eines erfindungsgemäßen Polymers oder Blends in einer PLED, insbesondere als Elektrolumineszenzmaterial.

Gegenstand der Erfindung ist ebenfalls eine PLED mit einer oder mehreren aktiven Schichten, wobei mindestens eine dieser aktiven Schichten ein oder mehrere erfindungsgemäße Polymere oder Blends enthält. Die aktive Schicht kann beispielsweise eine lichtemittierende Schicht und/oder eine Transportschicht und/oder eine Ladungsinjektionsschicht sein.

Die erfindungsgemäßen Polymere weisen gegenüber den in WO 03/020790 beschriebenen Poly-Spirobifluorenen, die über die 2,7-Positionen im Polymer verknüpft sind, und gegenüber Poly-Spirobifluorenen gemäß WO 97/20877 und EP 0882082, die über die 2,2'-Positionen verknüpft sind, die hier als nächstliegender Stand der Technik genannt werden, folgende überraschenden Vorteile auf:
(1) Die Lebensdauer ist höher als bei vergleichbaren Polymeren, die bei ähnlicher Zusammensetzung keine Einheiten gemäß Formel (1) enthalten, sondern statt dessen Spirobifluorene, die über die 2,7- oder insbesondere über die 2,2'-Positionen verknüpft sind. Eine Verbesserung der Lebensdauer ist für die Anwendung von enormer Bedeutung, da insbesondere bei blau und grün emittierenden Polymeren die unzureichende Lebensdauer bislang noch das größte Hindernis für eine technische Anwendung ist. Dieses Ergebnis ist überraschend gegenüber konjugierten Polymeren auf Basis von 2,7-verknüpften Spirobifluorenen, da man bislang meist davon ausgegangen war, dass eine hohe Konjugation eine notwendige Voraussetzung für gute Lebensdauer ist. Die bessere Lebensdauer gegenüber Polymeren, die 2,2'-verknüpfte Spirobifluoren-Einheiten enthalten, ist ebenfalls unerwartet, da auch diese Polymere teilkonjugiert sind und ähnliche Einheiten enthalten.
(2) Die erfindungsgemäßen Polymere weisen, bei ansonsten gleicher Zusammensetzung, vergleichbare oder höhere Leuchteffizienzen in der Anwendung auf. Dies ist von enormer Bedeutung, da somit entweder gleiche Helligkeit bei geringerem Energieverbrauch erzielt werden kann, was vor allem bei mobilen Applikationen (Displays für Handys, Pager, PDA, etc.), die auf Batterien und Akkus angewiesen sind, sehr wichtig ist. Umgekehrt erhält man bei gleichem Energieverbrauch höhere Helligkeiten, was beispielsweise für Beleuchtungsanwendungen interessant sein kann.
(3) Insbesondere im Vergleich zu 2,7-verknüpften Spirobifluoren-Polymeren ist eine tiefer blaue Emissionsfarbe mit einer schärferen Emissionsbande erzielbar. Dies lässt sich möglicherweise mit der definierten und kürzeren Konjugationslänge im Vergleich zu vollständig konjugierten Polymeren erklären. Daher sind die erfindungsgemäßen Polymere besser geeignet als Polymere gemäß dem Stand der Technik zur Einpolymerisation eines anderen blauen Emitters, da dadurch die Energie besser auf den Emitter übertragen werden kann.

Im vorliegenden Anmeldetext und auch in den im Weiteren folgenden Beispielen wird auf die Verwendung der erfindungsgemäßen Polymere in Bezug auf PLEDs und entsprechende Displays abgezielt. Trotz dieser Beschränkung der Beschreibung ist es für den Fachmann ohne weiteres erfinderisches Zutun möglich, die erfindungsgemäßen Polymere als Halbleiter (oder bei geeigneter Dotierung auch Leiter) auch für weitere Verwendungen in anderen elektronischen, Devices (Vorrichtungen) zu benutzen, z. B. in organischen Feld-Effekt-Transistoren (O-FETs), organischen integrierten Schaltungen (O-ICs), organischen Dünnfilmtransistoren (O-TFTs), organischen Solarzellen (O-SCs), organischen Feld-Quench-Devices (O-FQD, z. B. gemäß M. Redecker *et al., Proc. 22*^{*nd*} *International Display Research Conf.,* Nice 2002, S. 97-99) oder auch organischen Laserdioden (O-Laser), um nur einige Anwendungen zu nennen. Die Verwendung der erfindungsgemäßen Polymere in den entsprechenden Vorrichtungen ist ebenfalls ein Gegenstand der vorliegenden Erfindung. Weiterhin Gegenstand der Erfindung sind organische Feld-Effekt-Transistoren (O-FETs), organische integrierte Schaltungen (O-ICs), organische Dünnfilmtransistoren (O-TFTs), organische Solarzellen (O-SCs), organische Feld-Quench-Devices (O-FQDs) und organische Laserdioden (O-Laser), enthaltend mindestens ein erfindungsgemäßes Polymer.

Ebenso ist es für den Fachmann ein Leichtes, die oben gemachten Beschreibungen für Polymere ohne weiteres erfinderisches Zutun auf entsprechende Dendrimere oder Oligomere zu übertragen. Derartige Dendrimere und Oligomere sind ebenfalls ein Gegenstand der vorliegenden Erfindung.

### Beispiele:

### Beispiel 1: Synthese von Monomeren, die in Polymeren zu Einheiten gemäß Formel (1) führen

Die Synthese von 4,4'-Dibrom-1,1'-dimethoxy-spirobifluoren (Monomer **M1**) erfolgte gemäß der Literatur (X. Cheng *et al., Org. Lett.* **2004,** *6*, 2381-2383).

### Beispiel 2: Synthese weiterer Monomere

Die Synthese der Monomere **M2** bis **M5** ist in WO 03/020790 und der darin zitierten Literatur beschrieben. Die Synthese von 2,2'-Dibromspirobifluoren (Monomer **M6**), das für das Vergleichspolymer verwendet wurde, wurde gemäß F.-I. Wu *et al.* (*J. Mater. Chem.* **2002,** *12,* 2893-2897) durchgeführt. Die Strukturen der weiteren Monomere für erfindungsgemäße Polymere und Vergleichspolymere sind im Folgenden abgebildet.

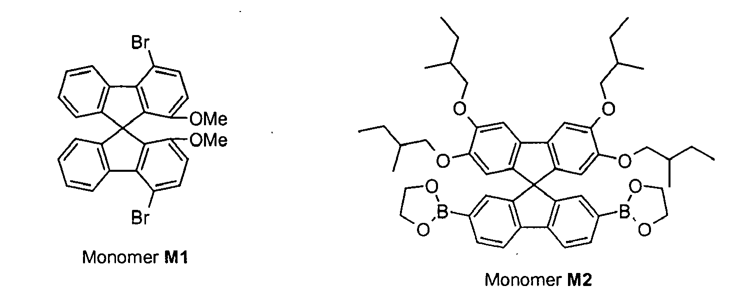

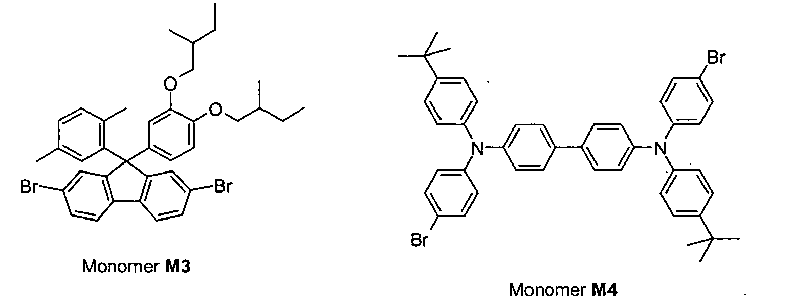

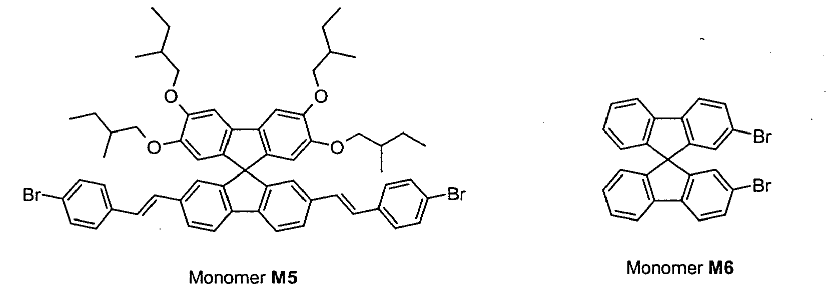

### Beispiel 3: Synthese der Polymere

Die Polymere wurden durch SUZUKI-Kupplung gemäß WO 03/048225 synthetisiert. Die Zusammensetzung der synthetisierten Polymere **P1** bis **P3** ist in Tabelle 1 zusammengefasst. Außerdem wurden die Vergleichspolymere **V1** und **V2** synthetisiert, die statt des Monomers **M1,** das im Polymer zu Einheiten gemäß Formel (1) führt, das Monomer **M3** bzw. das Monomer **M6** enthalten. Die Zusammensetzung dieser Vergleichspolymere ist ebenfalls in Tabelle 1 aufgeführt.

### Beispiel 4: Herstellung der PLEDs

Die Polymere wurden für einen Einsatz in PLEDs untersucht. Die PLEDs waren jeweils Zweischichtsysteme, d. h. Substrat//ITO//PEDOT//Polymer//Kathode. PEDOT ist ein Polythiophen-Derivat (Baytron P, von H. C. Starck, Goslar). Als Kathode wurde in allen Fällen Ba/Ag (Aldrich) verwendet. Wie PLEDs dargestellt werden können, ist in WO 04/037887 und der darin zitierten Literatur ausführlich beschrieben.

### Beispiel 5 bis 9: Device-Beispiele

Die Ergebnisse, die bei Verwendung der Polymere **P1** bis **P3** in PLEDs erhalten wurden, sind in Tabelle 1 zusammengefasst. Ebenso aufgeführt sind die Elektrolumineszenz-Ergebnisse, die unter Verwendung der Vergleichspolymere **V1** und **V2** erhalten wurden.

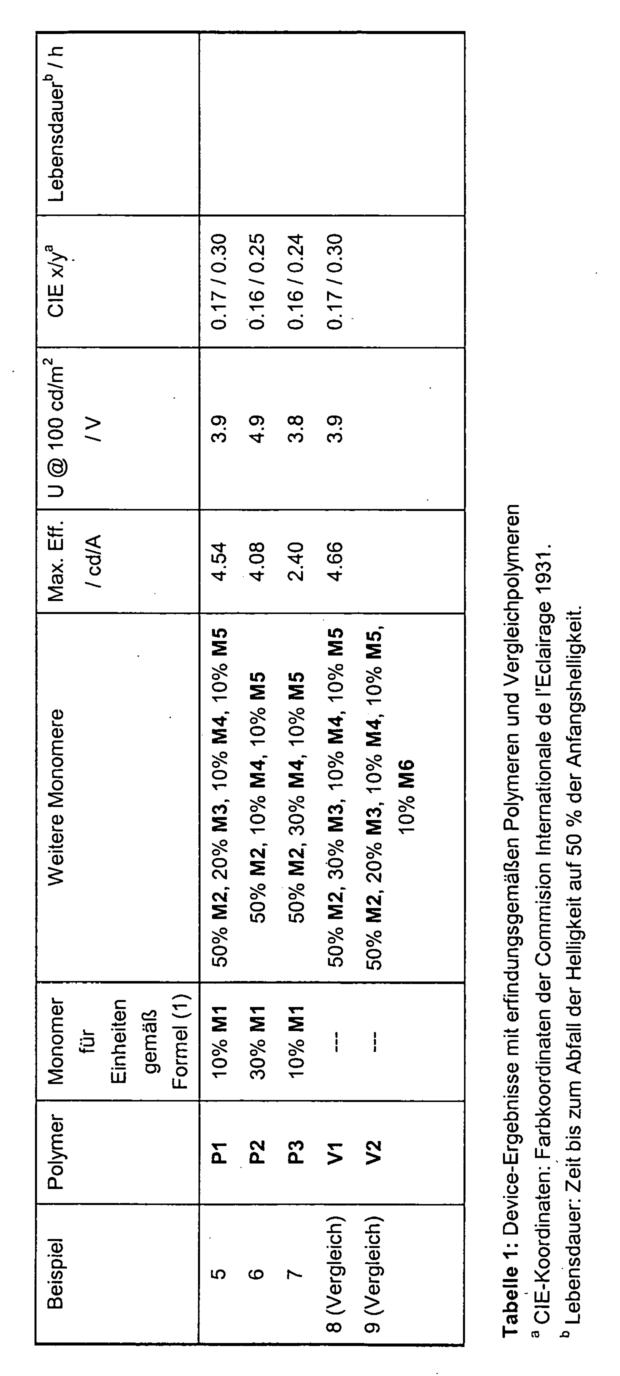

## Patentansprüche

1. Polymere, enthaltend mindestens 1 mol% einer ersten Wiederholeinheit gemäß Formel (1), 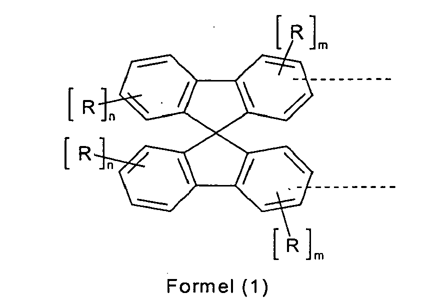 wobei die verwendeten Symbole und Indizes die folgende Bedeutung besitzen:
R ist bei jedem Auftreten gleich oder verschieden F, Cl, Br, I, CN, NO₂ OH, N(R¹)₂, Si(R¹)₃, B(R¹)₂, eine geradkettige, verzweigte oder cyclische Alkyl-, Alkoxy- oder Thioalkoxygruppe mit 1 bis 40 C-Atomen, in der auch ein oder mehrere nicht benachbarte C-Atome durch -CR¹=CR¹-, -C≡C-, -NR¹-, -O-, -S-, -CO-O- oder -O-CO-O- ersetzt sein können und wobei auch ein oder mehrere H-Atome durch Fluor ersetzt sein können, ein aromatisches oder heteroaromatisches Ringsystem mit 2 bis 40 C-Atomen, welches auch durch einen oder mehrere nicht-aromatische Reste R substituiert sein kann; oder eine Kombination aus 2, 3, 4 oder 5 der oben genannten Gruppen; dabei können auch zwei oder mehrere der Reste R miteinander ein aliphatisches oder aromatisches, mono- oder polycyclisches Ringsystem bilden;
R¹ ist bei jedem Auftreten gleich oder verschieden H oder ein aliphatischer oder aromatischer Kohlenwasserstoffrest mit 1 bis 20 C-Atomen;
n ist bei jedem Auftreten gleich oder verschieden 0, 1, 2, 3 oder 4;
m ist bei jedem Auftreten gleich oder verschieden 0, 1, 2 oder 3;
die gestrichelten Bindungen deuten dabei die Verknüpfung im Polymer an; mit der Maßgabe, dass die Verknüpfung nicht über die 2,2'-Positionen erfolgt;
und enthaltend mindestens 1 mol% einer zweiten Wiederholeinheit, die entweder gleich einer Wiederholeinheit gemäß Formel (1) ist oder verschieden ist.

2. Polymere gemäß Anspruch 1, **dadurch gekennzeichnet, dass** für die Symbole und Indizes gilt:
R ist bei jedem Auftreten gleich oder verschieden F, CN, NO₂, OH, N(R¹)₂, B(R¹)₂, eine geradkettige, verzweigte oder cyclische Alkyl-, Alkoxy- oder Thioalkoxygruppe mit 1 bis 20 C-Atomen, in der auch ein oder mehrere nicht benachbarte C-Atome durch -CR¹=CR¹-, -C≡C-, -NR¹-, -O-, -S- oder -CO-O- ersetzt sein können und wobei auch ein oder mehrere H-Atome durch Fluor ersetzt sein können, ein aromatisches oder heteroaromatisches Ringsystem mit 3 bis 30 C-Atomen, welches auch durch einen oder mehrere nicht-aromatische Reste R substituiert sein kann; oder eine Kombination aus 2, 3 oder 4 der oben genannten Gruppen; dabei können auch zwei oder mehrere der Reste R miteinander ein aliphatisches oder aromatisches, mono- oder polycyclisches Ringsystem bilden;
R¹ ist wie in Anspruch 1 beschrieben;
n ist bei jedem Auftreten gleich oder verschieden 0, 1, 2 oder 3;
m ist bei jedem Auftreten gleich oder verschieden 0, 1 oder 2.

3. Polymere gemäß Anspruch 1 und/oder 2, **dadurch gekennzeichnet, dass** die Einheiten gemäß Formel (1) an den beiden Spiro-Molekülhälften symmetrisch substituiert sind.

4. Polymere gemäß einem oder mehreren der Ansprüche 1 bis 3, **dadurch gekennzeichnet, dass** die Verknüpfung der Einheiten gemäß Formel (1) über gleiche Positionen an den beiden Spiro-Molekülhälften erfolgt.

5. Polymere gemäß Anspruch 4, **dadurch gekennzeichnet, dass** die Verknüpfung der Einheiten gemäß Formel (1) über die 1,1'-Positionen oder über die 4,4'-Positionen erfolgt.

6. Polymere gemäß einem oder mehreren der Ansprüche 1 bis 5, **dadurch gekennzeichnet, dass** sie teilkonjugiert sind.

7. Polymere gemäß Anspruch 6, **dadurch gekennzeichnet, dass** das Polymer außer Einheiten gemäß Formel (1) keine weiteren Einheiten enthält, die die Konjugation des Polymers unterbrechen.

8. Polymere gemäß einem oder mehreren der Ansprüche 1 bis 7, **dadurch gekennzeichnet, dass** das Polymer neben Einheiten gemäß Formel (1) noch weitere Strukturelemente enthält.

9. Polymere gemäß Anspruch 8, **dadurch gekennzeichnet, dass** weitere Einheiten aromatische, carbocyclische Strukturen mit 6 bis 40 C-Atomen sind, die substituiert oder unsubstituiert sein können, wobei als Substituenten die in Anspruch 1 definierten Reste R in Frage kommen.

10. Polymere gemäß Anspruch 9, **dadurch gekennzeichnet, dass** die weiteren Einheiten ausgewählt sind aus der Gruppe der Fluoren-Derivate, der 2,7-Spirobifluoren-Derivate, der Dihydrophenanthren-Derivate, der cis- oder trans-Indenofluoren-Derivate, der 1,4-Phenylen-Derivate, der 4,4'-Biphenylylen-Derivate, der 4,4"-Terphenylylen-Derivate, der 2,7- oder 3,6-Phenanthren-Derivate, der Dihydropyren- und/oder Tetrahydropyren-Derivate.

11. Polymere gemäß einem oder mehreren der Ansprüche 8 bis 10, **dadurch gekennzeichnet, dass** weitere Strukturelemente solche sind, die die Morphologie und gegebenenfalls auch die Emissionsfarbe der resultierenden Polymere beeinflussen, die bevorzugt ausgewählt sind aus der Gruppe der durch R substituierten oder unsubstituierten kondensierten aromatischen Strukturen mit 6 bis 40 C-Atomen oder Tolan-, Stilben- oder Bisstyrylarylenderivate.

12. Polymere gemäß einem oder mehreren der Ansprüche 8 bis 11, **dadurch gekennzeichnet, dass** weitere Strukturelemente die Lochinjektions- und/oder Lochtransporteigenschaften der Polymere erhöhen und bevorzugt ausgewählt sind aus der Gruppe der aromatischen Amine oder Phosphine oder elektronenreichen Heterocyclen.

13. Polymere gemäß einem oder mehreren der Ansprüche 8 bis 12, **dadurch gekennzeichnet, dass** weitere Strukturelemente die Elektroneninjektions- und/oder Elektronentransporteigenschaften der Polymere erhöhen und bevorzugt ausgewählt sind aus der Gruppe der elektronenarmen Aromaten oder Heterocyclen oder der Triarylborane.

14. Polymere gemäß einem oder mehreren der Ansprüche 8 bis 13, **dadurch gekennzeichnet, dass** weitere Strukturelemente Einheiten gemäß Anspruch 12 und Einheiten gemäß Anspruch 13 aneinander gebunden enthalten.

15. Polymere gemäß einem oder mehreren der Ansprüche 8 bis 14, **dadurch gekennzeichnet, dass** weitere Strukturelemente solche sind, welche auch bei Raumtemperatur mit hoher Effizienz aus dem Triplettzustand Licht emittieren können.

16. Polymere gemäß Anspruch 15, **dadurch gekennzeichnet, dass** diese Strukturelemente Elemente der Gruppen 8 bis 10 (Ru, Os, Rh, Ir, Pd, Pt) enthalten.

17. Polymere gemäß Anspruch 15 und/oder 16, **dadurch gekennzeichnet, dass** weitere Strukturelemente anwesend sind, welche den Übergang vom Singulettzum Triplett-Zustand verbessern, bevorzugt ausgewählt aus der Gruppe der Carbazol-Derivate, der Keto-, Phosphinoxid-, Sulfoxid-, Sulfon- oder Silaneinheiten.

18. Polymere gemäß einem oder mehreren der Ansprüche 1 bis 17, **dadurch gekennzeichnet, dass** sie neben Einheiten gemäß Formel (1) mindestens 30 mol% Einheiten gemäß Anspruch 9 und/oder 10 enthalten.

19. Polymere gemäß einem oder mehreren der Ansprüche 1 bis 20, **dadurch gekennzeichnet, dass** sie neben Einheiten gemäß Formel (1) mindestens 5 mol% Einheiten gemäß Anspruch 13 und/oder 14, die die Ladungstransporteigenschaften verbessern, enthalten.

20. Polymere gemäß einem oder mehreren der Ansprüche 1 bis 19, **dadurch gekennzeichnet, dass** der Anteil an Einheiten gemäß Formel (1) zwischen 5 und 70 mol% liegt.

21. Polymere gemäß einem oder mehreren der Ansprüche 1 bis 20, **dadurch gekennzeichnet, dass** die Polymere statistische, alternierende oder blockartige Strukturen aufweisen oder auch mehrere dieser Strukturen abwechselnd besitzen und linear, verzweigt oder dendritisch aufgebaut sind.

22. Mischungen (Blends) enthaltend ein oder mehrere Polymere gemäß einem oder mehreren der Ansprüche 1 bis 21 zusammen mit weiteren polymeren, oligomeren, dendritischen oder niedermolekularen Substanzen.

23. Mischungen gemäß Anspruch 22, **dadurch gekennzeichnet, dass** die zugemischte Verbindung eine Verbindung ist, die bei Raumtemperatur aus dem Triplett-Zustand Licht emittieren kann.

24. Lösungen und Formulierungen aus einem oder mehreren Polymeren oder Blends gemäß einem oder mehreren der Ansprüche 1 bis 23 in einem oder mehreren Lösungsmitteln.

25. Bifunktionelle monomere Verbindungen gemäß Formel (2), 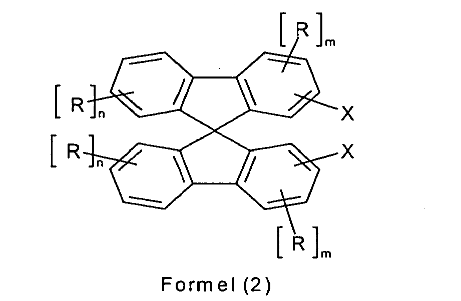 wobei R¹, n und m dieselbe Bedeutung haben, wie in Anspruch 1 beschrieben, und weiterhin gilt:
X ist bei jedem Auftreten gleich oder verschieden eine Gruppe, die unter Bedingungen der C-C- bzw. C-N-Verknüpfung copolymerisiert;
R ist bei jedem Auftreten gleich oder verschieden CN, NO₂, OH, N(R¹)₂, Si(R¹)₃, B(R¹)₂, eine geradkettige, verzweigte oder cyclische Alkyl-, Alkoxy-oder Thioalkoxygruppe mit 1 bis 40 C-Atomen, in der auch ein oder mehrere nicht benachbarte C-Atome durch -CR¹=CR¹-, -C≡C-, -NR¹-, -O-, -S-, -CO-O- oder -O-CO-O- ersetzt sein können und wobei auch ein oder mehrere H-Atome durch Fluor ersetzt sein können, ein aromatisches oder heteroaromatisches Ringsystem mit 2 bis 40 C-Atomen, welches auch durch einen oder mehrere nicht-aromatische Reste R substituiert sein kann; oder eine Kombination aus 2, 3, 4 oder 5 der oben genannten Gruppen; dabei können auch zwei oder mehrere der Reste R miteinander ein aliphatisches oder aromatisches, mono- oder polycyclisches Ringsystem bilden;
wobei die folgende Verbindung von der Erfindung ausgeschlossen ist: 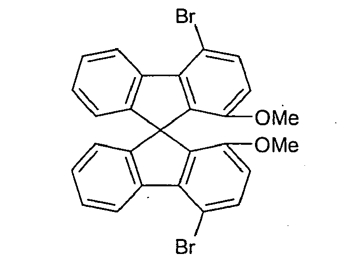 und weiterhin mit der Maßgabe, dass die beiden Gruppen X nicht in 2 und 2'-Position gebunden sind.

26. Bifunktionelle monomere Verbindungen gemäß Anspruch 24, **dadurch gekennzeichnet, dass** X ausgesucht ist aus der Gruppe besehend aus Cl, Br; I, O-Tosylat, O-Triflat, O-SO₂R¹, B(OR¹)₂ und Sn(R¹)₃, wobei R¹ dieselbe Bedeutung hat, wie in Anspruch 1 beschrieben, und wobei zwei oder mehr Reste R¹ auch miteinander ein Ringsystem bilden können.

27. Verwendung eines Polymers oder Blends gemäß einem oder mehreren der Ansprüche 1 bis 24 in einer organischen elektronischen Vorrichtung, insbesondere als Elektrolumineszenzmaterial.

28. Organische elektronische Vorrichtung mit einer oder mehreren aktiven Schichten, wobei mindestens eine dieser aktiven Schichten ein oder mehrere Polymere oder Blends gemäß einem oder mehreren der Ansprüche 1 bis 23 enthält.

29. Organische elektronische Vorrichtung gemäß Anspruch 28, **dadurch gekennzeichnet, dass** es sich um eine polymere Leuchtdiode (PLED), einen organischen Feld-Effekt-Transistor (O-FET), eine organische integrierte Schaltung (O-IC), einen organischen Dünnfilmtransistor (O-TFT), eine organische Solarzelle (O-SC), ein organisches Feld-Quench-Device (O-FQD) oder eine organische Laserdiode (O-Laser) handelt.
